# EUROPÄISCHE PATENTSCHRIFT

(11) **EP 3 378 389 B1**
(45) Veröffentlichungstag und Bekanntmachung des Hinweises auf die Patenterteilung: **21.08.2024**
(21) Anmeldenummer: 17162005.7
(22) Anmeldetag: 21.03.2017
(51) Int. Cl.: A61B 5/05

(54) **VORRICHTUNG FÜR BILDGEBENDE VERFAHREN AUF BASIS DES MAGNETIC PARTICLE IMAGING UND ZUGEHÖRIGE VERFAHREN**
DEVICE FOR IMAGING METHODS BASED ON MAGNETIC PARTICLE IMAGING AND RELATED METHODS
DISPOSITIF DE FORMATION D'IMAGE SUR LA BASE D'UNE IMAGERIE MAGNÉTOSCOPIQUE ET PROCÉDÉ ASSOCIÉ

(43) Veröffentlichungstag der Anmeldung: 26.09.2018
(73) Patentinhaber: Universität zu Lübeck, 23538 Lübeck (DE)
(72) Erfinder: Medimagh, Hanne, 38104 Braunschweig (DE); Gräser, Matthias, 23554 Lübeck (DE); Bringout, Gael, 76275 Ettlingen (DE); Weber, Matthias, 23552 Lübeck (DE); Buzug, Thorsten M., 23627 Groß Sarau (DE)
(74) Vertreter: RCD-Patent Giesen, Schmelcher & Griebel Patentwanwälte PartG mbB

(56) Entgegenhaltungen:
- WO-A2-2009/104151
- JP-A- 2009 195 614
- US-A1- 2008 309 330
- US-A1- 2010 249 578
- US-A1- 2015 008 910
- US-A1- 2016 296 145

## Beschreibung

Die Erfindung betrifft eine Vorrichtung für bildgebende Verfahren auf Basis des Magnetic Particle Imaging und zugehöriges Verfahren.

### Hintergrund

Bei bisher bekannten Verfahren des Magnetic Particle Imaging (MPI) werden lokale Konzentrationen magnetisierbarer Nanopartikel in einer a priori unbekannten räumlichen Verteilung im Innern eines Objekts ermittelt.

Beispielsweise können super-paramagnetische Partikel, insbesondere Eisenoxidpartikel, in einer Untersuchungsregion durch ein mit vorbestimmter Frequenz periodisch veränderliches Magnetfeld, nachfolgend auch als Anregungsfeld (engl. "drive field") bezeichnet, periodisch magnetisiert werden, wobei die Magnetisierung der Partikel nichtlinear von der Gesamtmagnetfeldstärke abhängt. Erfasst und analysiert man das Zeitverhalten der Partikel-Magnetisierung z.B. mit Hilfe von Detektionsspulen, so weist das Mess-Signal höhere Harmonische der Frequenz des Anregungsfeldes auf. Mittels geeigneter Auswertungstechniken kann auf die Partikel-Konzentration geschlossen werden, z.B. mittels der Formulierung und Lösung eines inversen Problems mit Hilfe der sogenannten Systemfunktion, üblicherweise nach einer Fourier-Analyse des Mess-Signals, siehe hierzu beispielsweise WO 2014 / 005 658 A1, Seite 2 bis Seite 4. Alternativ hierzu kann die Bildrekonstruktion auch direkt im Zeitbereich ohne die Formulierung und Lösung eines inversen Problems erfolgen, indem das induzierte Spannungssignal fortlaufend gemessen und direkt einem bestimmtem Punkt im Bild zugeordnet wird (sogenanntes x-Space-Verfahren, siehe z.B. "On the formulation of the image reconstruction problem in magnetic particle imaging", Mandy Grüttner et al., in Biomedizinische Technik/Biomedical Engineering 58(6):583-591, 2013, DOI: 10.1515/bmt-2012-0063).

In bisherigen Systemen wird zur Eingrenzung kleiner Volumina der Untersuchungsregion das Anregungsfeld mit einem - oft zeitlich konstanten - Selektionsmagnetfeld (engl. "selection field") überlagert. Das Selektionsmagnetfeld weist dann an wenigstens einem vorbestimmten Punkt der Untersuchungsregion eine Nullstelle auf. Ausgehend von diesem so genannten feldfreien Punkt, kurz: FFP, steigt das Selektionsmagnetfeld in alle Richtungen schnell an, so dass magnetisierbare Nanopartikel schon in geringem Abstand zum FFP in die magnetische Sättigung gelangen. Weit vom FFP entfernte Nanopartikel reagieren dann kaum noch auf das Anregungsfeld und leisten keinen signifikanten Beitrag zum detektierten Signal. Das MPI-Signal stammt vielmehr aus der lokalen Umgebung des FFP und gibt über die dort vorhandene lokale Partikel-Konzentration Auskunft. Zur Klarstellung sei an dieser Stelle erwähnt, dass zur Erzeugung von MPI-Signal nicht unbedingt eine exakte Nullstelle des Selektionsmagnetfeldes benötigt wird; ein Bereich mit niedriger Feldamplitude des Selektionsmagnetfeldes reicht dafür auch.

Aus der US Patentanmeldung US 2008 / 0 309 330 A1 ist ein MPI Gerät bekannt. Bei diesem Gerät kann der zu untersuchende Patient auf einer Liegeplatziert werden, wobei in der Liege Empfangsspulen angeordnet sind, sodass der Patient zusammen mit den Empfangsspulen innerhalb der Anordnung verschoben werden kann.

Aus der internationalen Patentanmeldung WO 2009 / 104 151 A2 ist eine Anordnung bekannt, bei der ein medizinisches Instrument einen Teil eines MPI Gerätes darstellt. Das Gerät soll zur oberflächennahen Erkennung geeignet sein.

Aus der japanischen Patentanmeldung JP 2009 / 195 614 A ist ein der US Patentanmeldung US 2008 / 0 309 330 A1 ähnliches Gerät bekannt.

Mit Hilfe des Anregungsfeldes werden der FFP und der diesen umgebenden Bereich niedriger Gesamtmagnetfeldstärke innerhalb der Untersuchungsregion verschoben; der FFP durchläuft dabei eine Bahnkurve, nachfolgend auch als Trajektorie bezeichnet, die offen sein kann, in der Regel jedoch geschlossen ist, so dass der FFP nach einer vorbestimmten Repetitionszeit an seinen Ausgangspunkt zurückkehrt. Das Anregungsfeld ist zeitabhängig und wird üblicherweise von Elektromagneten erzeugt.

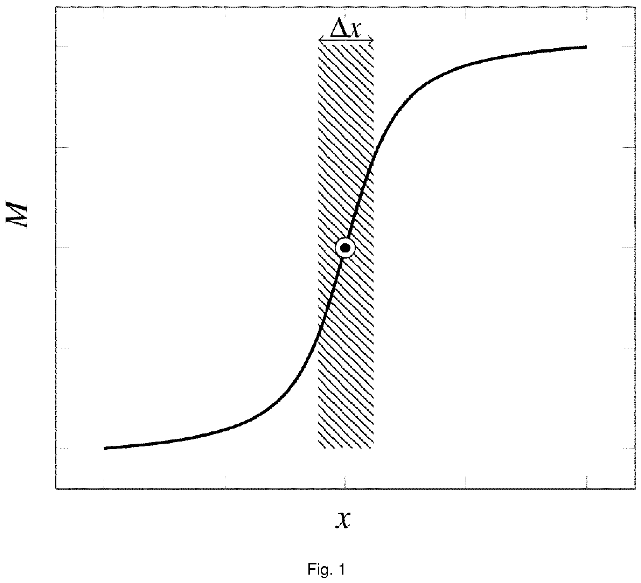

In Fig. 1 ist ein typischer Magnetisierungsverlauf von super-paramagnetischen Partikeln dargestellt, wobei in Fig. 1 die Gegenwart eines Selektionsgradienten angenommen ist, der eine Ortsabhängigkeit erzeugt.

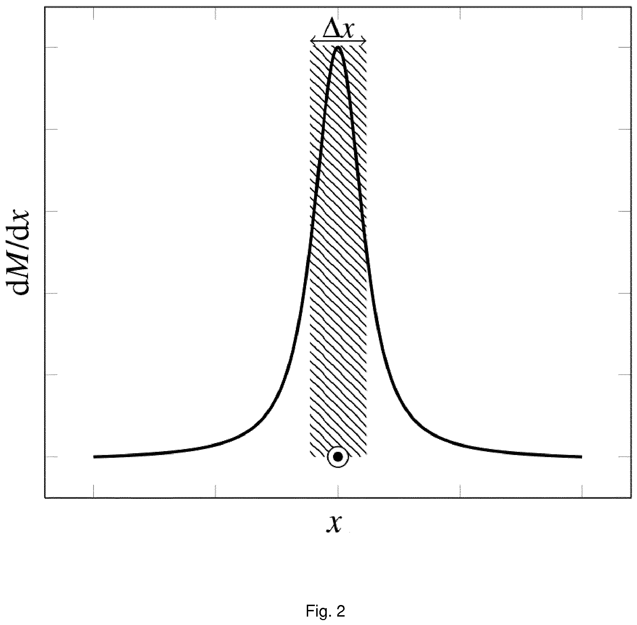

Unter der Wirkung eines Selektionsmagnetfeldes, welches bei x = 0 einen FFP mit einem Magnetfeldgradienten G einrichtet, zeigt die Magnetisierung eines Partikels näherungsweise den Verlauf einer Langevin-Funktion. In Fig. 1 befindet sich das Partikel nur innerhalb einer Umgebung der Breite Δx um den FFP nicht in der magnetischen Sättigung und antwortet auf das Anregungsfeld des MPI. Üblicherweise wird die Breite Δx mit der Peakbreite ("full width half maximum", FWHM) der Ableitung dM/dx gleichgesetzt, wie in Fig. 2 schraffiert dargestellt.

Wie man anhand von Fig. 1 und Fig. 2 erkennt, zieht ein höherer Magnetfeldgradient G im FFP auch einen steileren Verlauf der Langevin-Funktion durch x = 0 und somit einen schmaleren Peak (kleinere Peakbreite Δx) nach sich.

Unter dem "Field of View" (FOV) einer MPI-Mess-Vorrichtung, nachfolgend auch als MPI-Scanner bezeichnet, versteht man den Raumbereich, den ein FFP während des Verlaufs der Messung überstreichen kann.

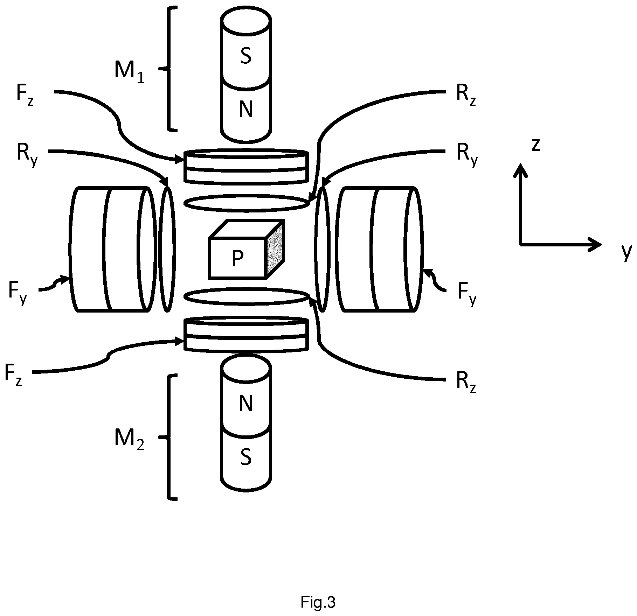

In der Praxis unterteilt man das FOV gewöhnlich in würfelförmige Voxel mit der Kantenlänge Δx (wie oben definiert) oder kleiner und tastet es wie oben beschrieben mit einer Trajektorie ab. Bekannt sind dabei ein-, zwei- und drei-dimensionale Abtastungen. Dabei ist ein typisches Abtastsystem aus Fig. 3 ersichtlich.

Hier wird mittels einer vorbestimmten Anordnung eine Probe P, die super-paramagnetische Partikel beinhaltet, mit verschiedenen Magnetfeldern beaufschlagt.

Beispielsweise werden über die als Permanentmagneten M₁ und M₂ dargestellten Magneten starke äußere Magnetfelder zugeführt, die so gestaltet sind, dass in der Probe P eine feldfreie Linie (FFL) / ein feldfreier Punkt (FFP) entsteht, d.h. die Permanentmagneten stellen das Selektionsfeld zur Verfügung. Eine FFL kann als eine Aneinanderreihung einer Vielzahl von FFPs entlang einer Linie aufgefasst werden.

Mittels der Spulenpaare F_{y} bzw. F_{z} können nun jeweils getrennt in y- oder z- Richtung durch gezielte Ansteuerung Anregungsfelder erzeugt werden, mit Hilfe derer die FFL / der FFP innerhalb der Probe P in z- bzw. y-Richtung verschoben werden kann. D.h. die Permanentmagneten M₁ und M₂ und die Spulenpaare F_{y} bzw. F_{z} stellen Vertreter von Felderzeugungseinrichtungen dar.

Mit Hilfe der zugeordneten Empfangsspule(npaare) R_{y} bzw. R_{z} kann dann das jeweilige Signal in y- bzw. z-Richtung ausgelesen werden. Die Empfangsspulen(paare) stellen Vertreter von Felddetektionseinrichtungen dar.

Ohne weiteres kann dieses System auch noch um eine dritte Dimension, in x-Richtung, erweitert werden.

Ist das zu untersuchende Volumen größer als durch die Austeuerung der Anregungsfelder aufgelöst werden kann, muss die Probe P innerhalb der Vorrichtung verschoben werden.

Zur Abtastung des Volumens können unterschiedliche Ansätze verwendet werden. Dabei können sowohl statische als auch oszillierende Magnetfelder zum Einsatz kommen.

In einer zukünftigen medizinischen Anwendung des MPI-Verfahrens wird man zur tomographischen Bildgebung beliebiger Körperteile oder auch des ganzen Körpers eines Patienten deutlich größere Spulenabstände realisieren müssen. Beispielsweise kann man davon ausgehen, dass die elektromagnetischen Spulen einen Abstand von etwa 0,5 m aufweisen müssen. Es sind dann relativ große Selektions- und Anregungsfeldamplituden zu erzeugen, damit über das gesamte FOV hinweg Magnetfeldgradienten der Größenordnung bis zu 1 T/m eingerichtet werden können. Der Patient wird während des Scanvorgangs insofern kontinuierliche Magnetfeldänderungen um die Größenordnungen 0,1 bis 1 T in seinem Körper erdulden müssen. Dies kann unerwünschte Nebenwirkungen haben. Beispielsweise ist aus Reilly, J.P., "Magnetic field excitation of peripheral nerves and the heart: a comparison of thresholds", Med. Bio. Engin. Comp., 29 (6), 571-579, 1991 bekannt, dass zeitlich veränderliche Magnetfelder zu wahrnehmbaren - vereinzelt auch schmerzhaften - peripheren Nervenstimulationen (PNS) führen können. Verantwortlich dafür ist die Induktion elektrischer Feldgradienten im Bereich der Nervenfasern durch magnetische Flussdichteänderungen dB/dt. Reilly gibt insbesondere Schwellenwerte zum Auslösen der PNS für die Flussdichteamplituden sinusförmig oszillierender homogener Magnetfelder an, die ihrerseits von der Oszillationsfrequenz abhängen. Gemäß Literaturauffassung beschränkt der Amplitudenbereich 0,1 bis 1 T in einem humanen MPI-Scanner bzw. 3D-Anregungsspulenset den sicheren - d.h. zuverlässig PNS-freien - Bereich auf Feldfrequenzen im Subkilohertzbereich, wenn sich während der Anregungszeit weder die Person noch die Spulen bewegen.

Während bisherige MPI-Scanner ein FOV mit einem Volumen deutlich kleiner als 1 dm³ aufweisen, müssen zukünftige medizinische MPI-Scanner mit einem FOV-Volumen im Bereich 10 bis 100 dm³ oder mehr ausgestattet sein. Zudem können sie in einem humanen Ganzkörper-Scanner voraussichtlich nur periodische Anregungsfelder verwenden, die - wie oben beschrieben - Frequenzen im Subkilohertzbereich aufweisen. Beides zusammen wird aber auf eine deutliche Erhöhung der Repetitionszeiten von FFP- oder FFL-Trajektorien führen und somit erzwingen, dass eine ausreichend dichte Abtastung des FOV für ein - ggf. menschengroßes - Objekt eine Zeitspanne benötigt, die 1 bis 2 Größenordnungen über denen der heutigen Laborsysteme liegt.

Insbesondere einem lebenden Patienten ist eine sehr lange Verweildauer in einem MPI-Scanner wohl kaum zumutbar. Weiterhin müsste der Patient während der gesamten Verweildauer ruhig liegen bleiben, womit sowohl die Anwendbarkeit für bestimmte Körperregionen ausgeschlossen ist, oder aber wegen einer nötigen Sedierung des Patienten eine weitere Belastung mit möglichen weiteren Komplikationen zu erwarten ist. Überdies sind lange Mess-Zeiten schon aus Gesichtspunkten der Nutzungsökonomie des MPI-Scanners von Nachteil.

Für verschiedene Anwendungsszenarien stehen Scannersysteme mit unterschiedlichen Spulentopologien zur Verfügung. Dabei kann grob zwischen sogenannten geschlossenen Systemen mit einer zylinderförmigen Patientenöffnung, offenen Systemen mit lateralem Patientenzugang und sogenannten "SingleSided" Systemen mit uneingeschränktem Patientenzugang unterschieden werden.

Sämtliche bisher vorgestellten Scannersysteme haben jedoch den Nachteil, dass es nur stark eingeschränkte Möglichkeiten gibt, die lokale Signalqualität zu erhöhen.

Ein Signal von hoher Qualität wird als ein Signal verstanden, aus dem ein Bild mit hoher Qualität (hohes Signal-zu-Rausch-Verhältnis (signal-to-noise ratio, SNR) und/oder hohe Auflösung) rekonstruiert werden kann. Beim systemfunktionsbasierten MPI benötigt man dafür ein Signal mit hohem SNR, das eine Systemfunktion mit einer guten Konditionierung (niedrige Konditionszahl, d.h. geringe Rauschverstärkung) liefert.

Die Erzeugung des Selektionsfeldes ist außerdem mit erheblichem technischem und finanziellem Aufwand verbunden, der insbesondere darin begründet ist, dass eine erhebliche elektrische Leistung für die Felderzeugung zur Verfügung gestellt werden muss, sodass gleichzeitig auch ein erhöhter Kühlaufwand betrieben werden muss.

Zudem wird die Feldberechnung bei der Gegenwart von weichmagnetischen Materialien kompliziert. Die Feldberechnung ist bei der systemfunktionsbasierten Bildrekonstruktion wichtig im Rahmen des Sequenzdesigns und bei der modellbasierten und/oder schnellen Bildrekonstruktion.

Zur Verbesserung der lokalen Signalqualität können zwar mittels einer geeigneten Wahl der Stromamplituden Bildfelder (Fields of View, FOVs) erzeugt werden, deren Position und Größe für die medizinische Diagnostik in der jeweiligen Fragestellung angepasst ist. Dieses Verfahren ist allerdings anfällig für sogenannte Trunkationsartefakte, die durch signalgebende Partikel außerhalb des Bildfeldes entstehen. Zur Erhöhung der Sensitivität bzw. des Signal-Rausch-Verhältnisses gibt es Ansätze in der Entwicklung optimierter Nanopartikel, in der Trajektorien- bzw. Sequenzoptimierung sowie in der Konstruktion von rauscharmen Empfangsketten für das MPI-System. Die Verwendung einer FFL erhöht im Vergleich zu einem FFP das SNR, löst aber nicht das zugrunde liegende Problem der Beschränkung des Ortsbereichs, aus dem das Signal stammt.

Die Ortskodierung mittels eines Selektionsfeldes mit einem FFP oder einer FFL führt dazu, dass jeweils nur ein kleiner Teil der Partikel zum Signal beiträgt (geringe Sensitivität bzw. geringes SNR). Dies gilt insbesondere bei einer hohen Gradientenstärke des Selektionsfeldes, die jedoch für eine hohe Ortsauflösung benötigt wird.

D.h. die bisherigen Ansätze führen zu einer gesteigerten Komplexität und / oder unbefriedigenden Fortschritten bei teilweise enormem Aufwand.

### Aufgabe

Es ist daher eine Aufgabe der Erfindung eine verbesserte Vorrichtung und ein verbessertes Verfahren bereitzustellen, die es erlauben die lokale Signalqualität im Magnetic Particle Imaging zu verbessern.

### Kurzdarstellung der Erfindung

Die Erfindung ist definiert in den unabhängigen Ansprüchen.

Weitere vorteilhafte Ausgestaltungen sind Gegenstand der abhängigen Ansprüche und der ausführlichen Beschreibung.

### Kurzdarstellung der Figuren

Nachfolgend wird die Erfindung näher unter Bezugnahme auf die Figuren erläutert werden. In diesen zeigt:
- Fig. 1: einen typischen räumlichen Magnetisierungsverlauf von super-paramagnetischen Partikeln, wobei die Gegenwart eines Selektionsgradienten angenommen ist, der eine Ortsabhängigkeit erzeugt,
- Fig. 2: die Ableitung des räumlichen Magnetisierungsverlaufes gemäß Fig. 1,
- Fig. 3: eine schematische Darstellung eines klassischen MPI-Scanners, und
- Fig. 4: eine schematische Darstellung einer Anordnung zur Ortung einer im Mess-Volumen befindlichen Spule gemäß einer Ausführungsform der Erfindung.

### Detaillierte Beschreibung

Nachfolgend wird die Erfindung eingehender unter Bezugnahme auf die Figuren dargestellt werden. Dabei ist anzumerken, dass unterschiedliche Aspekte beschrieben werden, die jeweils einzeln oder in Kombination zum Einsatz kommen können. D.h. jeglicher Aspekt kann mit unterschiedlichen Ausführungsformen der Erfindung verwendet werden, soweit nicht explizit als reine Alternative dargestellt.

Weiterhin wird nachfolgend der Einfachheit halber in aller Regel immer nur auf eine Entität Bezug genommen werden. Soweit nicht explizit vermerkt, kann die Erfindung aber auch jeweils mehrere der betroffenen Entitäten aufweisen. Insofern ist die Verwendung der Wörter "ein", "eine" und "eines" nur als Hinweis darauf zu verstehen, dass in einer einfachen Ausführungsform zumindest eine Entität verwendet wird.

Die Erfindung macht sich die Erkenntnis zu Nutze, dass für eine angestrebte Erhöhung der lokalen Signalqualität unter anderem die Tatsache problematisch ist, dass insbesondere die Felddetektionseinrichtungen relativ zu den Felderzeugungseinrichtungen (und damit bei jeder beliebigen statischen Probenpositionierung auch relativ zur Probe P) fix waren.

Die Erfindung ermöglicht nunmehr eine flexible Anordnung sowohl der Felderzeugungseinrichtungen als auch der Felddetektionseinrichtungen.

Felderzeugungseinrichtungen und Felddetektionseinrichtungen, die flexibel in ihrer Position, Orientierung und / oder Form (bzw. anderen relevanten Eigenschaften) sind, erlauben im Gegensatz zu bisherigen Systemen eine flexible Erhöhung der lokalen Signal- bzw. Bildqualität in genau dem Ortsbereich, der für die medizinische Diagnostik in der jeweiligen Fragestellung relevant ist.

Dabei macht sich die Erfindung zu Nutze, dass die Sensitivität mit der Entfernung abnimmt, d.h. die Sensitivität ist aufgrund des Biot-Savart'schen Gesetzes in der unmittelbaren Nähe eines Leiters bzw. einer Spule als Felddetektionseinrichtung am Größten.

Zusätzlich kann die Signalqualität auch im Sinne einer hohen lokalen Unterschiedlichkeit der Signale gesteigert werden.

Einerseits kann mit flexiblen Felddetektionseinrichtungen klassische MPI-Bildgebung unter Verwendung eines statischen Selektionsfelds betrieben werden, wobei die Bildgebung durch die flexiblen Felddetektionseinrichtungen unterstützt wird (Steigerung der lokalen Sensitivität und Signalqualität).

Andererseits ist mit Hilfe von flexiblen Felddetektionseinrichtungen auch eine Bildgebung ganz ohne ein Selektionsfeld möglich. Die Ortskodierung wird in dem Fall z.B. über die Veränderung der Empfangsspuleneigenschaften realisiert werden, d.h. über eine Bewegung (Verschiebung/Drehung) der Empfangsspulen gegenüber ortsfesten Sendespulen und / oder über Verformung. In diesem Fall würde also der klassische Bildgebungsprozess "invertiert": Anstatt ein örtlich beschränktes Signal mit homogener Sensitivität zu erfassen, würde z.B. ein homogen angeregter Bereich durch zeitlich variable Sensitivitäts-Profile abgetastet, wodurch insgesamt mehr Signal erzeugt und erfasst wird.

Dieser Ansatz erlaubt eine beschleunigte Bildgebung.

Die Erfindung sieht daher vor eine Vorrichtung für bildgebende Verfahren auf Basis des Magnetic Particle Imaging bereitzustellen. Die Vorrichtung weist eine magnetische Felderzeugungseinrichtung auf, wobei die magnetische Felderzeugungseinrichtung ein magnetisches Aussteuerungsfeld in einem zu untersuchenden Bereich erzeugt.

Dabei kann die Felderzeugungseinrichtung eine statische Felderzeugungseinrichtung und / oder eine dynamische Felderzeugungseinrichtung enthalten. Die Felderzeugungseinrichtung muss dabei auch nicht ortsfest sein, sondern kann auch vorbestimmt in Bezug auf ihre Lage verändert werden. Eine Änderung der Lage kann sich auch virtuell daraus ergeben, dass einzelne räumlich unterschiedliche Felderzeugungseinrichtungen in einer vorbestimmten zeitlichen Abfolge verwendet werden.

Weiterhin weist die Vorrichtung zumindest eine Felddetektionseinrichtung zur Detektion von Magnetfeldern auf, wobei die zumindest eine Felddetektionseinrichtung in Bezug auf ihre räumliche Position und / oder Orientierung und / oder in Bezug auf ihre intrinsischen elektro-magnetischen Eigenschaften zwischen zwei Detektionen bzw. während einer Detektion veränderlich ist.

D.h. die vorgestellte Vorrichtung ist hoch flexibel und kann in unterschiedlichster Weise zu einer lokalen Verbesserung der Signalqualität führen.

Beispielhaft kann dies z.B. so verwendet werden, dass die Felddetektionseinrichtung mittels geeigneter Vorrichtungen in den Körper eines Menschen oder Tieres eingebracht wird und zwar möglichst nahe an das zu untersuchende Gebiet.

Beispielhafte Techniken sind z.B. Katheter, Trokar oder Biopsienadeln, mit denen eine erfindungsgemäße Empfangsspule als eine beispielhafte Felddetektionseinrichtung verbunden werden kann und an den zu untersuchenden Ort gebracht werden kann.

Das Feld wird mittels der Felderzeugungseinrichtungen erzeugt, wobei aber nunmehr wegen der räumlichen Nähe der Felddetektionseinrichtung zum zu untersuchenden Volumen die Bildqualität erheblich höher ist.

Beispielsweise kann die Felddetektionseinrichtung in ein Lumen, wie z.B. Ösophagus, Arterien, Venen, Urethra oder das Rektum eingebracht werden und somit beispielsweise eine intra-abdominale MPI-Bildgebung mit hoher Sensitivität ermöglichen. Beispielsweise kann dies zu Erkennung von benignem oder malignem Gewebe, z.B. in der Prostata, Niere, Blase, Leber, Galle, Speicheldrüse, Magen, Lunge verwendet werden, die bisher nicht oder nur unzureichend mit bisherigen Techniken dargestellt werden konnten.

Hierdurch wird eine Erhöhung der Bildqualität in genau dem Bereich ermöglicht, in dem z.B. eine Intervention durchgeführt werden soll bzw. der von besonderem Interesse ist. So können z.B. arteriosklerotische Plaques charakterisiert werden oder aber Gefäßstenosen quantifiziert werden oder gutartige / bösartige Gewebeveränderungen detektiert werden.

Erfindungsgemäß ist zumindest eine Felddetektionseinrichtung in Bezug auf ihre räumliche Position und Orientierung und in Bezug auf ihre intrinsischen elektro-magnetischen Eigenschaften zwischen zwei Detektionen bzw. während einer Detektion veränderlich.

Die Bereitstellung der erfindungsgemäßen veränderlichen Felddetektionseinrichtung schließt jedoch nicht aus, dass zusätzlich auch klassische Felddetektionseinrichtungen wie in Fig. 3 zusätzlich zur Verwendung kommen.

Zudem erlaubt die Erfindung nunmehr auch auf ein Selektionsfeld zu verzichten, da z.B. durch eine Bewegung der Felddetektionseinrichtung in gleicher Weise eine Ortsauflösung erzielbar ist.

Natürlich erlaubt die Erfindung auch, dass eine Ortsauflösung sowohl über das Bewegen der Felddetektionseinrichtung als auch zusätzlich über ein Selektionsfeld ermöglicht wird. Hierbei können insbesondere die unterschiedlichen Charakteristika der beiden Effekte dazu genutzt werden Daten bezüglich bestimmter Voxel mittels verschiedener Techniken zu erlangen, z.B. mittels eines Nah-Scans, bei dem die Felddetektionseinrichtung nahe am zu untersuchenden Voxel befindlich ist, und eines Fern-Scans, bei dem mittels des Selektionsfeldes das zu untersuchende Voxel in den Fokus gerückt wird.

Dabei sei angemerkt, dass die Änderung der intrinsischen elektro-magnetischen Eigenschaften insbesondere eine Änderung von Spuleneigenschaften umfasst, wie sie z.B. durch die Hinzuschaltung weiterer Spulenabschnitte, Änderung der Spulengeometrie, insbesondere der Spulenlänge und / oder Spulendicke erreicht werden kann. Beispielsweise kann die Spulenlänge durch geeignete mechanische Elemente verkürzt oder verlängert werden. Erfindungsgemäß beinhaltet die Spule oder ist umgeben von thermisch aktivierbarem Memory-Material, sodass durch eine geeignete Steuerung die intrinsischen elektro-magnetischen Eigenschaften geändert werden können. D.h. es kann nicht nur das Sensitivitätsprofil einer Spule geändert werden, sondern auch das Empfangsverhalten einer Spule insgesamt.

Wie bereits angemerkt, kann es von Vorteil sein, dass die Position der Felddetektionseinrichtung getrennt erfasst werden kann, sodass der Vorgang der Positionierung / Orientierung der Felddetektionseinrichtung nicht vorbekannt sein muss, sondern autark erfasst werden kann.

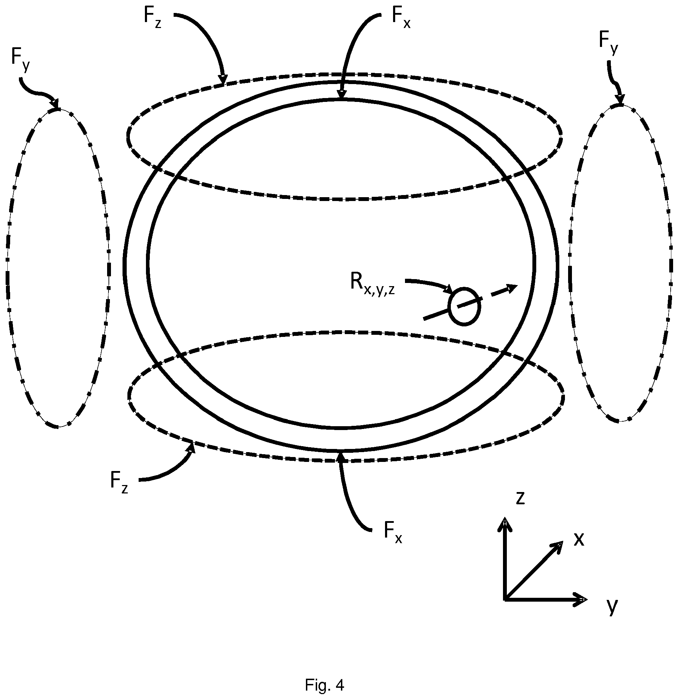

Beispielsweise kann hierfür vorgesehen sein, dass die räumliche Position und / oder Orientierung durch eine Anregung der Felddetektionseinrichtung und Detektion erkannt wird. D.h. wie in Fig. 4 aufgezeigt kann nunmehr die Felddetektionseinrichtung invertierend als Felderzeugungseinrichtung betrieben werden und die für eine MPI-Messung zu verwendenden Felderzeugungseinrichtungen könnten nun als Felddetektionseinrichtungen verwendet werden. Eine "Felddetektionseinrichtung" R_{x,y,z} wird z.B. mit einer oder mehreren Frequenzen (in einer vorbestimmten Weise) angeregt und zum Abstrahlen von elektro-magnetischer Strahlung angeregt. Mit anderen Worten, der "Felddetektionseinrichtung" R_{x,y,z} wird ein Sendesignal bekannter Frequenz aufgeprägt. In einem homogenen 3D-Ortungs-Empfangsspulenset Fₓ, F_{y}, F_{z} wird eine Spannung gleicher Frequenz gemessen, welche durch ein Lock-in Verfahren zudem sehr sensitiv bestimmt werden kann. Aus dem Amplitudenverhältnis der drei Spulenpaare Fₓ, F_{y}, F_{z} kann die genaue Orientierung errechnet werden. Werden zudem die Spannungen in den einzelnen Spulen separat ausgelesen, kann durch das inhomogene Empfangsverhalten und die bereits bekannte Orientierung eine genaue Position der Spule im Mess-Volumen bestimmt werden. In die Berechnung gehen dabei die bekannten Sensitivitätsprofile aller Spulen, die bekannte Sequenz des applizierten Stroms und die in den äußeren Spulen gemessenen Spannungssignale ein.

Ohne Beschränkung der Allgemeinheit kann natürlich die Ortung der Felddetektionseinrichtung R_{x,y,z} auch mit getrennten Spulen oder gänzlich anderen Positionsbestimmungstechniken bewerkstelligt werden. Die Kombination in einem invertierten Betrieb würde jedoch eine vorteilhafte räumlich kompakte Integration erlauben.

Wie bereits zuvor beschrieben kann die Erfindung sowohl mit als auch ohne Selektionsfeld verwendet werden. Daher weist die Vorrichtung vorteilhafter Weise eine Einrichtung zur Erzeugung eines Selektionsfeldes auf, wobei diese wie aus dem Stand der Technik bekannt gestaltet sein kann.

In einer Weiterbildung der Erfindung weist die Vorrichtung nicht nur eine Felddetektionseinrichtung, sondern eine Vielzahl von Felddetektionseinrichtungen auf. Hierdurch können zu einem bestimmten Zeitpunkt eine Vielzahl von räumlich unterschiedlichen Punkten gleichzeitig untersucht bzw. deren Signale erfasst werden, wodurch z.B. der Mess-Zeitraum verkürzt werden kann.

Zudem ist es möglich, dass zumindest ein Teil der Felddetektionseinrichtungen eine andere Sensitivität als Felddetektionseinrichtungen eines anderen Teils aufweisen. Hierdurch können unterschiedliche Sensitivitätsprofile parallel zum Einsatz kommen und so zu einer Verbesserung des Datenmaterials führen, z.B. eine Verbesserung des Kontrastes und / oder der Auflösung.

Durch die flexible Gestaltung der Vorrichtung ist es nunmehr möglich die Felddetektionseinrichtung R_{x,y,z} auch innerhalb des zu untersuchenden Bereiches zu variieren, sodass mit einer hohen Präzision nicht nur eine Verschiebung parallel zu den Felddetektionseinrichtungen Fₓ, F_{y}, F_{z}, sondern auch in jedem beliebigen Winkel zu den Achsen der Felddetektionseinrichtungen Fₓ, F_{y}, F_{z} ermöglicht wird.

Aber auch, wenn die Felddetektionseinrichtung R_{x,y,z} außerhalb des zu untersuchenden Bereiches platziert ist, d.h. z.B. räumlich benachbart, kann mittels der Felddetektionseinrichtung R_{x,y,z} sinnvoll gemessen werden, sodass auch hier mittels einer Veränderung weitere Vorteile erzielt werden können.

Wie bereits zuvor beschrieben kann die Felddetektionseinrichtung R_{x,y,z} so dimensioniert werden, dass sie mittels eines Katheters in ein Lumen oder in eine Körperöffnung oder mittels einer Biopsienadel in den Körper eines Patienten eingebracht werden kann.

Mittels der vorgestellten Vorrichtung können auch erfindungsgemäße Verfahren zur Verwendung kommen.

Insbesondere weist ein erfindungsgemäßes nichtinvasives bildgebendes Verfahren auf Basis des Magnetic Particle Imaging mittels einer zuvor beschriebenen Vorrichtung zumindest die nachfolgenden Schritte auf.

Mittels der Vorrichtung wird ein erster Datensatz gemessen, wobei die zumindest eine Felddetektionseinrichtung R_{x,y,z} eine erste räumliche Position und erste Orientierung und erste intrinsische elektro-magnetische Eigenschaften aufweist. Datensatz ist dabei weit zu verstehen und kann Daten einer oder mehrerer Felddetektionseinrichtungen, als auch Daten mit oder ohne (statischem oder dynamischem) Selektionsfeld beinhalten. Nach Abschluss der Messung wird die zumindest eine Felddetektionseinrichtung R_{x,y,z} in Bezug auf ihre räumliche Position und / oder Orientierung und / oder in Bezug auf ihre intrinsischen elektro-magnetischen Eigenschaften verändert. Die Veränderung ist entweder vorbekannt oder wird wie bereits zuvor beschrieben geeignet erfasst. Es wird nach der Veränderung ein zweiter Datensatz gemessen, wobei die zumindest eine Felddetektionseinrichtung eine veränderte räumliche Position und / oder eine veränderte Orientierung und / oder veränderte intrinsische elektro-magnetische Eigenschaften aufweist. Anschließend werden sowohl der erste als auch der zweite Datensatz getrennt ausgewertet.

Ohne weiteres kann aber alternativ ein erfindungsgemäßes nichtinvasives bildgebendes Verfahren auf Basis des Magnetic Particle Imaging mittels einer zuvor beschriebenen Vorrichtung zumindest die nachfolgenden Schritte aufweisen. Mittels der Vorrichtung wird ein erster Datensatz gemessen, anschließend wird die Felddetektionseinrichtung R_{x,y,z} in Bezug auf ihre räumliche Position und / oder Orientierung und / oder in Bezug auf ihre intrinsischen elektro-magnetischen Eigenschaften verändert und ein zweiter Datensatz gemessen. Anschließend werden die Datensätze zusammen ausgewertet. Im Unterschied zum vorherigen Verfahren wird die Felddetektionseinrichtung R_{x,y,z} während der Gesamtmessung verändert.

In einer Weiterbildung der Verfahren wird nach Abschluss der Messung eines ersten Datensatzes die zumindest eine Felddetektionseinrichtung R_{x,y,z} in Bezug auf ihre räumliche Position und / oder Orientierung und / oder in Bezug auf ihre intrinsischen elektro-magnetischen Eigenschaften verändert und anschließend ein zweiter Datensatz gemessen.

Ohne weiteres kann aber alternativ ein erfindungsgemäßes nichtinvasives bildgebendes Verfahren auf Basis des Magnetic Particle Imaging mittels einer zuvor beschriebenen Vorrichtung zumindest die nachfolgenden Schritte aufweisen. Während mittels der Vorrichtung ein erster Datensatz gemessen wird, wird die Felddetektionseinrichtung R_{x,y,z} in Bezug auf ihre räumliche Position und / oder Orientierung und / oder in Bezug auf ihre intrinsischen elektro-magnetischen Eigenschaften verändert.

Mittels der Erfindung können also in flexibler Weise sowohl während der Messung als auch bei wiederholten Messungen die Eigenschaften der zumindest einen Felddetektionseinrichtung R_{x,y,z} in Bezug auf ihre räumliche Position und / oder Orientierung und / oder in Bezug auf ihre intrinsischen elektro-magnetischen Eigenschaften verändert werden und so eine hohe Vielfalt unterschiedlicher Messprotokolle realisiert werden. Insofern kann sich ein Datensatz auf eine Folge von Messungen als auch eine einzige Messung beziehen. Dementsprechend kann ein Messprotokoll eine Anzahl von unterschiedlichen Messungen vorsehen, wobei z.B. innerhalb einer Messung zumindest eine Felddetektionseinrichtung R_{x,y,z} in Bezug auf ihre räumliche Position und / oder Orientierung und / oder in Bezug auf ihre intrinsischen elektro-magnetischen Eigenschaften verändert wird als auch alternativ oder zusätzlich zwischen einzelnen Messungen zumindest eine Felddetektionseinrichtung R_{x,y,z} in Bezug auf ihre räumliche Position und / oder Orientierung und / oder in Bezug auf ihre intrinsischen elektro-magnetischen Eigenschaften verändert werden.

D.h. mit den vorgestellten Vorrichtungen und Verfahren ist es nunmehr möglich die Signalqualität im Magnetic Particle Imaging zu verbessern. Insbesondere ist es möglich mittels der Erfindung die lokale Signalqualität zu erhöhen bzw. die Sensitivität bzw. das Signal-Rausch-Verhältnis zu erhöhen und / oder die bisherige Komplexität und die damit verbundenen Kosten zu reduzieren.

Mit der Erfindung ist es möglich die Bewegung der Felddetektionseinrichtung relativ zur Felderzeugungseinrichtung (während einer Einzelmessung oder zwischen Einzelmessungen) bereitzustellen.

D.h. mit der Abkehr von den etablierten Paradigmen der Spulen mit unveränderlichen Eigenschaften als auch der Ermöglichung der Invertierung des Mess-Szenarios kann eine erhebliche Steigerung des Signal-Rausch-Verhältnisses bzw. der Signalqualität erreicht werden.

## Patentansprüche

1. Vorrichtung für bildgebende Verfahren auf Basis des Magnetic Particle Imaging, aufweisend
• eine magnetische Felderzeugungseinrichtung, wobei die magnetische Felderzeugungseinrichtung ein magnetisches Aussteuerungsfeld in einem zu untersuchenden Bereich erzeugt, und
• zumindest eine Felddetektionseinrichtung zur Detektion von Magnetfeldern,
• wobei die zumindest eine Felddetektionseinrichtung in Bezug auf ihre räumliche Position und Orientierung und in Bezug auf ihre intrinsischen elektro-magnetischen Eigenschaften zwischen zwei Detektionen bzw. während einer Detektion veränderlich ist,
• wobei die Spulen in Bezug auf intrinsische elektro-magnetische Eigenschaften veränderlich sind, die Spule thermisch aktivierbarem Memory-Material beinhaltet oder die Spule von thermisch aktivierbarem Memory-Material umgeben ist, sodass durch eine geeignete Steuerung die intrinsischen elektro-magnetischen Eigenschaften geändert werden können.

2. Vorrichtung nach Anspruch 1, **dadurch gekennzeichnet, dass** die räumliche Position und / oder Orientierung durch eine Anregung der Felddetektionseinrichtung und Detektion erkannt wird.

3. Vorrichtung nach Anspruch 2, **dadurch gekennzeichnet, dass** die räumliche Position und / oder Orientierung durch einen invertierten Betrieb mittels Anregung der Felddetektionseinrichtung mit einer oder mehreren Frequenzen und Detektion von abgestrahlter elektro-magnetischer Strahlung mittels der Felderzeugungseinrichtungen erkannt wird.

4. Vorrichtung nach Anspruch 2 oder 3, **dadurch gekennzeichnet, dass** zumindest Teile der Felderzeugungseinrichtung so betrieben werden, dass Feld hervorgerufen durch die Anregung der Felddetektionseinrichtung erkannt und in Bezug auf räumliche Position und / oder Orientierung der Felddetektionseinrichtung verarbeitet wird.

5. Vorrichtung nach Anspruch 4, **dadurch gekennzeichnet, dass** aus der Orientierung von Spulenpaaren der Felderzeugungseinrichtung und der gemessenen Spannungen die Position der Felddetektionseinrichtung im Mess-Volumen bestimmt wird.

6. Vorrichtung nach einem der vorhergehenden Ansprüche, **dadurch gekennzeichnet, dass** die Vorrichtung weiterhin eine Einrichtung zur Erzeugung eines Selektionsfeldes aufweist.

7. Vorrichtung nach einem der vorhergehenden Ansprüche, **dadurch gekennzeichnet, dass** die räumliche Position und / oder Orientierung der Felddetektionseinrichtung in dem zu untersuchenden Bereich veränderlich ist.

8. Vorrichtung nach einem der vorhergehenden Ansprüche, **dadurch gekennzeichnet, dass** die Felddetektionseinrichtung außerhalb des zu untersuchenden Bereich räumlich veränderlich ist.

9. Vorrichtung nach einem der vorhergehenden Ansprüche, **dadurch gekennzeichnet, dass** die Felddetektionseinrichtung auf
• einen Katheter
oder
• einer Biopsienadel angeordnet ist, um in den Körper
eines Patienten eingebracht zu werden.

10. Nichtinvasives bildgebendes Verfahren auf Basis des Magnetic Particle Imaging während einer Gesamtmessung verändert mittels einer Vorrichtung nach einem der vorhergehenden Ansprüche, aufweisend die Schritte:
• Messen eines ersten Datensatzes, wobei die zumindest eine Felddetektionseinrichtung eine erste räumliche Position und erste Orientierung und erste intrinsische elektro-magnetische Eigenschaften aufweist,
• Verändern der zumindest eine Felddetektionseinrichtung in Bezug auf ihre räumliche Position und / oder Orientierung und / oder in Bezug auf ihre intrinsischen elektro-magnetischen Eigenschaften,
• Messen eines zweiten Datensatzes, wobei die zumindest eine Felddetektionseinrichtung eine veränderte räumliche Position und / oder eine veränderte Orientierung und / oder veränderte intrinsischen elektro-magnetischen Eigenschaften aufweist,
• Auswerten des ersten und zweiten Datensatzes.

11. Nichtinvasives bildgebendes Verfahren auf Basis des Magnetic Particle Imaging mittels einer Vorrichtung nach einem der vorhergehenden Ansprüche, aufweisend die Schritte:
• Messen eines ersten Datensatzes,
• Verändern der zumindest eine Felddetektionseinrichtung in Bezug auf ihre räumliche Position und / oder Orientierung und / oder in Bezug auf ihre intrinsischen elektro-magnetischen Eigenschaften,
• Auswerten des ersten Datensatzes.

12. Verfahren nach Anspruch 11, **dadurch gekennzeichnet, dass** nach Abschluss der Messung eines ersten Datensatzes die zumindest eine Felddetektionseinrichtung in Bezug auf ihre räumliche Position und / oder Orientierung und / oder in Bezug auf ihre intrinsischen elektro-magnetischen Eigenschaften verändert wird, und anschließend ein zweiter Datensatz gemessen wird.

13. Verfahren nach Anspruch 11 oder 12, **dadurch gekennzeichnet, dass** während der Messung eines ersten Datensatzes die zumindest eine Felddetektionseinrichtung in Bezug auf ihre räumliche Position und / oder Orientierung und / oder in Bezug auf ihre intrinsischen elektro-magnetischen Eigenschaften verändert wird.

## Claims

1. A device for imaging methods based on magnetic particle imaging, comprising
• a magnetic field generation device, wherein the magnetic field generation device generates a magnetic modulation field in a region to be analysed, and
• at least one field detection device for detecting magnetic fields,
• wherein the at least one field detection device is changeable in respect of its spatial position and/or orientation and/or in respect of its intrinsic electromagnetic properties between two detections or during a single detection,
• wherein the coils are changeable in respect of intrinsic electromagnetic properties, the coil contains thermally activatable memory material or the coil is surrounded by thermally activatable memory material so that the intrinsic electromagnetic properties can be changed using a suitable control system.

2. The device according to claim 1, **characterised in that** the spatial position and/or orientation is detected by excitation of the field detection device and detection.

3. The device according to claim 2, **characterised in that** the spatial position and/or orientation by inverted operation by exciting the field detection device with one or more frequencies and detecting emitted electromagnetic radiation by means of the field generation devices.

4. The device according to claim 2 or 3, **characterised in that** at least parts of the field generation device are operated in such a way that field caused by the excitation of the field detection device is detected and processed in relation to the spatial position and/or orientation of the field detection device.

5. The device according to claim 4, **characterised in that** the position of the field detection device in the measurement volume is determined from the orientation of coil pairs of the field generation device and the measured voltages.

6. The device according to one of the preceding claims, **characterised in that** the device further comprises a device for generating a selection field.

7. The device according to one of the preceding claims, **characterised in that** the spatial position and/or orientation of the field detection device in the area to be examined is changeable.

8. The device according to one of the preceding claims, **characterised in that** the field detection device is spatially changeable outside the area to be examined.

9. The device according to one of the preceding claims, **characterised in that** the field detection device is arranged on
• a catheter
or
• a biopsy needle
in order to be introduced into the body of a patient.

10. A non-invasive imaging method based on magnetic particle imaging that changes during an overall measurement by means of a device according to one of the preceding claims, comprising the steps of:
• measuring a first data set, wherein the at least one field detection device has a first spatial position and first orientation and first intrinsic electromagnetic properties,
• changing the at least one field detection device in respect of its spatial position and/or orientation and/or in respect of its intrinsic electromagnetic properties,
• measuring a second data set, wherein the at least one field detection device has a changed spatial position and/or a changed orientation and/or changed intrinsic electromagnetic properties,
• analysing the first and second data set.

11. A non-invasive imaging method based on Magnetic Particle Imaging by means of a device according to any one of the preceding claims, comprising the steps of:
• measuring a first data set,
• changing the at least one field detection device in respect of its spatial position and/or orientation and/or in respect of its intrinsic electromagnetic properties,
• analysing the first data set.

12. The method according to claim 11, **characterised in that**, after completion of the measurement of a first data set, the at least one field detection device is changed in respect of its spatial position and/or orientation and/or in respect of its intrinsic electromagnetic properties, and a second data set is then measured.

13. The method according to claim 11 or 12, **characterised in that**, during the measurement of a first data set, the at least one field detection device is changed in respect of its spatial position and/or orientation and/or in respect of its intrinsic electromagnetic properties.

## Revendications

1. Dispositif pour un procédé délivrant des images sur la base de l'imagerie par particules magnétiques (**IPM**) comportant :
• un système de production de champ magnétique, sachant que le système de production de champ magnétique produit un champ de modulation magnétique dans un zone à analyser, et
• au moins un système de détection de champ pour la détection de champs magnétiques,
• sachant qu'au moins un système de détection de champ est modifiable eu égard à sa position et/ou orientation spatiale et eu égard à ses propriétés électromagnétiques intrinsèques entre deux détections ou pendant une détection,
• sachant que les bobines peuvent être modifiées eu égard aux propriétés électromagnétiques intrinsèques, la bobine contient un matériau de mémorisation pouvant être thermiquement activé ou la bobine est entourée d'un matériau de mémorisation pouvant être thermiquement activé de telle manière que les propriétés électromagnétiques intrinsèques peuvent être modifiées par une commandez appropriée.

2. Dispositif selon la revendication 1, **caractérisé en ce que** la position et/ou l'orientation spatiale sont identifiées par une excitation du système de détection de champ et une détection.

3. Dispositif selon la revendication 2, **caractérisé en ce que** la position et/ou l'orientation spatiale sont identifiées par un fonctionnement inversé au moyen de l'excitation du système de détection de champ avec une ou plusieurs fréquences et la détection de l'irradiation électromagnétique émise au moyen des systèmes de production de champ.

4. Dispositif selon la revendication 2 ou 3, **caractérisé en ce qu'**au moins des parties du système de production de champ sont utilisées de telle manière que le champ provoqué par l'excitation du système de détection de champ est identifié et est élargi en référence à la position et/ou orientation spatiale du système de détection de champ.

5. Dispositif selon la revendication 4, **caractérisé en ce que** la position du système de détection de champ est déterminée en volume de mesure à partir de l'orientation des paires de bobines du système de production de champ et des tensions mesurées.

6. Dispositif selon l'une quelconque des revendications précédentes, **caractérisé en ce que** le dispositif comporte en plus un système destiné à produire un champ de sélection.

7. Dispositif selon l'une quelconque des revendications précédentes, **caractérisé en ce que** la position et/ou l'orientation spatiale du système de détection de champ est modifiable dans la zone à analyser.

8. Dispositif selon l'une quelconque des revendications précédentes, **caractérisé en ce que** le système de détection de champ est modifiable dans l'espace en dehors de la zone à analyser.

9. Dispositif selon l'une quelconque des revendications précédentes, **caractérisé en ce que** le système de détection de champ est disposé sur
• un cathéter
ou
• une aiguille de biopsie pour être introduit dans le corps d'un patient.

10. Procédé non invasif délivrant des images sur la base de l'imagerie par particules magnétiques (**IPM**) pendant une mesure générale, modifié au moyen d'un dispositif selon l'une quelconque des revendications précédentes, comportant les étapes de :
• mesure d'un premier ensemble de données, sachant qu'au moins un système de détection de champ comporte une première position et/ou orientation spatiale et des premières propriétés électromagnétiques intrinsèques,
• modification d'au moins un système de détection de champ eu égard à sa position et/ou orientation spatiale et/ou eu égard à ses propriétés électromagnétiques intrinsèques,
• mesure d'un deuxième ensemble de données, sachant qu'au moins un système de détection de champ comporte une position spatiale modifiée et/ou une orientation spatiale modifiée et/ou des propriétés électromagnétiques intrinsèques modifiées,
• exploitation du premier et du deuxième ensemble de données.

11. Procédé non invasif délivrant des images imagerie non invasif sur la base de l'imagerie par particules magnétiques (**IPM**) au moyen d'un dispositif selon l'une quelconque des revendications précédentes, comportant les étapes de :
• mesure d'un premier ensemble de données,
• modification d'au moins un système de détection de champ eu égard à sa position et/ou orientation spatiale et/ou eu égard à ses propriétés électromagnétiques intrinsèques,
• exploitation du premier ensemble de données.

12. Procédé selon la revendication 11, **caractérisé en ce qu'**une fois la mesure terminée d'un premier ensemble de données, au moins un système de détection de champ est modifié eu égard à sa position et/ou orientation spatiale et/ou eu égard à ses propriétés électromagnétiques intrinsèques et un deuxième ensemble de données et ensuite mesuré.

13. Procédé selon la revendication 11 ou 12, **caractérisé en ce que** pendant la mesure d'un ensemble de données, au moins un système de détection de champ est modifié eu égard à sa position et/ou orientation spatiale et/ou eu égard à ses propriétés électromagnétiques intrinsèques.
